# EUROPÄISCHE PATENTANMELDUNG

(11) **EP 0 680 928 A1**
(43) Veröffentlichungstag der Anmeldung: **08.11.1995**
(21) Anmeldenummer: 95105826.2
(22) Anmeldetag: 19.04.1995
(51) Int. Cl.: C01B 21/084, C07D 213/61

(54) **Verfahren zur kontinuierlichen Herstellung von Nitrosylchlorid**

(30) Priorität: 02.05.1994 DE 4415337
(71) Anmelder: BAYER AG, 51368 Leverkusen (DE)
(72) Erfinder: Kraus, Helmut, Dr., D-51519 Odenthal (DE); Klausener, Alexander, Dr., D-50670 Köln (DE); Landscheidt, Heinz, Dr., D-47057 Duisburg (DE)

(57) **Zusammenfassung**

Die Reaktion von Stickstoffdioxid mit Chlorwasserstoff zu Nitrosylchlorid und Salpetersäure läßt sich vorteilhaft in einem Reaktor im Gleichstrom so durchführen, daß die Reaktanden unten eingeführt werden, das Nitrosylchlorid am Kopf und die Salpetersäure am Fuß des Reaktors entnommen werden.

## Beschreibung

Die Erfindung betrifft ein kontinuierliches Verfahren zur Herstellung von chlorfreiem Nitrosylchlorid aus gasförmigem Stickstoffdioxid und gasförmigem Chlorwasserstoff. Der Begriff "Stockstoffdioxid" im Sinne der Erfindung schließt das damit im Gleichgewicht stehende Distickstofftetroxid ein.

Nitrosylchlorid ist in der organischen Chemie eine häufig verwandte NO^{⊕}-Quelle, beispielsweise für Oximierungen, Nitrosierungen und Diazotierungen. Für die Herstellung von Nitrosylchlorid sind zahlreiche Verfahren beschrieben worden.

Die bekannteste Herstellungsmethode für Nitrosylchlorid ist die Zubereitung von Königswasser, wobei allerdings auch Chlor entsteht:

3 HCl + HNO₃ → NOCl + Cl₂ + H₂O

Einleiten von Chlorwasserstoff in eine Lösung von Nitrosylschwefelsäure in Schwefelsäure führt zur Bildung von Nitrosylchlorid nach der Gleichung

NOHSO₄ + HCl → NOCl + H₂SO₄

Die Methode führt bei fortschreitender Reaktion zu immer geringerer Umsetzung, so daß Nitrosylchlorid/Chlorwasserstoff-Gemische mit einem ständig fallenden Nitrosylchlorid-Anteil entstehen. Die Entsorgung der als Nebenprodukte anfallenden Chlorid- und Stickoxid-haltigen Schwefelsäure ist technisch aufwendig.

Einige andere Verfahren gehen von Stickstoffmonoxid aus: Gemäß DE-AS 1 169 903 wird die Umsetzung mit Chlor in Gegenwart eines Katalysators entsprechend der Gleichung 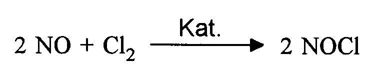
empfohlen. Nach US-PS 2 366 518 verläuft die Umsetzung mit gasförmigem Chlorwasserstoff in Gegenwart von Sauerstoff über Distickstofftrioxid:

2 NO + 1/2 O₂ → N₂O₃

N₂O₃ + 2 HCl → 2 NOCl + H₂O.

Zu wasserfreiem Nitrosylchlorid kommt man laut Comptes Rend. 204 (1932), 697 bis 699 durch Umsetzung von Distickstofftrioxid mit Thionylchlorid:

N₂O₃ + SOCl₂ → 2 NOCl + SO₂

Die genannten Verfahren sind mit dem Nachteil verbunden, daß sie das technisch schlecht verfügbare Stickstoffmonoxid als Ausgangsprodukt benötigen.

Die Bildung von Nitrosylchlorid aus Alkylnitriten in salzarmem Medium macht man sich beispielsweise bei der Diazotierung zunutze:

RONO + HCl ⇄ NOCl + ROH

C₆H₅ - NH₂ + NOCl → C₆H₅ -N₂^{⊕} Cl^{⊖}

Allerdings kann der mitentstandene Alkohol über eine Sandmeyer-Reaktion zu unerwünschten Nebenprodukten führen. Darüber hinaus werden Alkylnitrite meistens aus Natriumnitrit, Schwefelsäure und Alkohol hergestellt; für eine Herstellung in technischem Maßstab ist diese Methode wegen des hohen Salzanfalls, also nicht besonders gut geeignet.

Andere Verfahren zur Herstellung von Nitrosylchlorid gehen von technisch gut verfügbarem Stickstoffdioxid aus. Gemäß GB-PS 786 740 und FR-PS 1 333 767 leitet man Stickstoffdioxid in angefeuchtetes Alkali- oder Erdalkalichlorid und erhält allerdings als Nebenprodukt große Mengen Alkali- bzw. Erdalkalinitrat. Nach J. Chem. Phys. 18 (1950), 1411 entsteht dabei ein Gemisch aus Nitrosylchlorid, Nitroxylchlorid und Stickstoffdioxid.

Bei der Umsetzung von Stickstoffdioxid mit gasförmigem Chlorwasserstoff entsteht nach J. Amer. Chem. Soc. 63 (1942), 2520 als Nebenprodukt Chlor nach der Gleichung:

NO₂ + 2 HCl → NOCl + H₂O + 1/2 Cl₂

Laut US-PS 2 366 518 entsteht dabei wegen Nebenproduktbildung (Nitroxylchlorid, Salpetersäure, Stickoxide) nur wenig Nitrosylchlorid.

Gemäß DE-OS 2 019 216 wird Nitrosylchlorid durch Umsetzung von flüssigem Stickstoffdioxid und gasförmigem Chlorwasserstoff in Salpetersäure hergestellt und muß anschließend diskontinuierlich isoliert werden.

Die Aufgaben der Erfindung bestand in der Bereitstellung eines kontinuierlich ausführbaren Verfahrens, ausgehend von technich gut verfügbarem Stickstoffdioxid, zur Herstellung von Nitrosylchlorid in guter Ausbeute und in einer Reinheit, die es erlaubt, das entstandene Nitrosylchlorid ohne weiteren Reinigungsschritt zur über die Diazotierung laufenden Chlorierung von Aminoheterocyclen, z.B. Aminopyridinen und -pyrazolen, einzusetzen unter Vermeidung von schwer abtrennbaren und schwierig zu entsorgenden Nebenprodukten, wie Chlor, Stickoxiden und insbesondere großen Salzmengen, wobei die anfallende Salpetersäure unter Vermeidung zusätzlicher Reinigungsmaßnahmen in einer für weitere Verwendungen ausreichenden Qualität anfallen soll.

Es wurde nun überrraschenderweise gefunden, daß bei geeigneter Anordnung Stickstoffdioxid und Chlorwasserstoff in der Gasphase nahezu quantitativ zu Nitrosylchlorid hoher Reinheit umgesetzt werden können.

Gegenstand der Erfindung ist also ein Verfahren zur Herstellung von Nitrosylchlorid aus Chlorwasserstoff und Stickstoffdioxid im molaren Verhältnis von 0.4 bis 1, vorzugsweise 0.4 bis 0,8, insbesondere 0.5 bis 0,7, bei Temperaturen von 0 bis 100, vorzugsweise 0 bis 60°C, wonach man die Reaktanden im Gleichstrom von unten nach oben durch einen Reaktor führt und das gebildete Nitrosylchlorid oben und die gebildete Salpetersäure unten am Reaktor entnimmt.

Die Reaktanden können als solche oder in Mischung mit Inertgasen eingesetzt werden. Solche Inertgase umfassen beispielsweise Stickstoff, Kohlendioxid, Argon und die anderen Edelgase. Ein solches Inertgas kann getrennt oder mit einem oder beiden Reaktanden vorgemischt in den Reaktor eingespeist werden. Das Volumenverhältnis Inertgas/Stickstoffdioxid kann beispielsweise 0 bis 15, vorzugsweise 0.1 bis 8 betragen. Die Mitverwendung von Wasser ist für das erfindungsgemäße Verfahren nicht notwendig; geringe Mengen können jedoch zur Verdünnung der anfallenden Salpetersäure vorteilhaft sein.

Überschüssiger Chlorwasserstoff kann nach Kondensation des Nitrosylchlorids, gegebenenfalls zusammen mit dem Inertgas, entfernt und in die Reaktion zurückgeführt werden.

Das erfindungsgemäße Verfahren läßt sich grundsätzlich sowohl bei Normaldruck als auch bei vermindertem oder erhöhtem Druck durchführen. Als Druckbereich kommt der von 0,5 bis 10, bevorzugt 0,7 bis 7, besonders bevorzugt 1 bis 5 bar in Frage.

Für das erfindungsgemäße Verfahren zu verwendende Reaktoren und die Reaktionsbedingungen werden so gewählt, daß eine für eine vollständige Umsetzung ausreichende Verweilzeit gewährleistet wird.

Geeignete Reaktoren umfassen beispielsweise Reaktionskolonnen, die eine große Phasengrenzfläche (gasförmig/flüssig) und eine innige Vermischung der flüssigen und der gasförmigen Phase gewährleisten. Dies kann durch den Einbau von Böden, wie Glockenböden, Sieblochböden, Ventilböden, Schlitzböden usw., wie sie für eine thermische Trennung üblich sind, durch Füllkörper aller Art, wie sie bei thermischen Trennoperationen üblich sind, oder durch die Ausrüstung der Kolonnen mit ungeordneten Füllkörpern aller Art oder mit geordneten Packungen aus Metall, Keramik, Kunststoff, Glas oder weiteren Materialien, die gegenüber den Reaktionspartnern des erfindungsgemäßen Verfahrens inert sind, erreicht werden. Solche Kolonnen mit Einbauten, Füllungen oder Packungen sowie die Füllungen und Packungen selbst sind handelsüblich und dem Fachmann bekannt.

Bevorzugte Reaktoren sind röhrenförmig gebaut und besitzen ein Länge/Durchmesser-Verhältnis von 10 bis 150, vorzugsweise von 20 bis 70. Besonders bevorzugte Reaktoren sind Füllkörperkolonnen, wie sie für fraktionierte Destillationen eingesetzt werden, in nicht-waagrechter, also in senkrechter oder auch schräger Anordnung.

Die optimale Größe der nötigen Phasengrenzfläche im Reaktor hängt auch von Temperatur und Gasmenge ab. Im allgemeinen führen aber Oberflächen/Volumen-Verhältnisse von 1 bis 20, vorzugsweise 5 bis 15 cm⁻¹ zu zufriedenstellenden Ergebnissen.

Die Belastung der Reaktoren, bezogen auf die gesamte eingespeiste Gasmenge (Stickstoffdioxid, Chlorwasserstoff und gegebenenfalls Inertgas), beträgt in der Regel 10 bis 3 000, bevorzugt 20 bis 2 000 und besonders bevorzugt 40 bis 1 000 Nl/l freien Volumens in der Reaktionskolonne pro Stunde (Nl = Liter unter Normalbedingungen).

Weiterer Gegenstand der Erfindung ist die Verwendung des erfindungsgemäß hergestellten Nitrosylchlorids bei über die Diazotierung laufenden Chlorierungen von Aminoheterocyclen wie Aminopyridinen und -pyrazolen.

Die Gasverhältnisse der nachfolgenden Beispiele beziehen sich auf das Volumen; Prozentangaben beziehen sich auf das Gewicht.

### Beispiele

### Verwendete Apparatur:

Eine Glaskolonne mit einer Länge von 80 cm und einem inneren Durchmesser von 2 cm wurde mit Glaskugeln eines Durchmessers von 3 mm gefüllt. Am unteren Ende wurden die Reaktanden eingeführt. Der Kopf der Kolonne wurde mit Trockeneis gekühlt; dort wurde das kondensierte Nitrosylchlorid abgenommen.

### Beispiel 1

In das untere Ende der auf 25°C temperierten Kolonne wurden 15 l/h eines Chlorwasserstoff/Stickstoff-Gemisches (15:85) und nach 10 Minuten 1 l/h Stickstoffdioxid einer Temperatur von 25°C eingeleitet.

25 ml DMF wurden in einem 500 ml-Kolben mit Chlorwasserstoff gesättigt. Nach Verdünnung mit 200 ml DMF und Abkühlen auf -10°C wurden 20 ml des hergestellten rohen NOCl zugesetzt. Bei 0°C tropfte man eine Lösung von 17,3 g 2-Amino-5-aminomethylpyridin in 100 ml DMF zu. Nach Aufwärmen auf Raumtemperatur und 1 h Nachrühren engte man am Rotationsverdampfer ein. Das Rohprodukt wurde mit 0,2 n Natronlauge neutralisiert und mit Dichlormethan extrahiert. Nach Trocknen mit wasserfreiem Natriumsulfat und Einengen am Rotationsverdampfer wurden 14,2 g Produkt erhalten, welches laut GC mit internem Standard 85,8 % an 2-Chlor-5-chlormethyl-pyridin (61,4 % d. Theorie) enthielt.

### Beispiel 2

Durch die Glaskolonne wurden 1 Stunde lang 20 l/h eines Chlorwasserstoff/Stickstoff-Gemischs (15:85) in eine Lösung von 2,6 g 94,5 %igen 2-Amiono-5-aminomethylpyridins in 70 ml DMF eingeleitet. Nach Abkühlung des Ansatzes auf 0°C wurden 1 Stunde 2 l/h Stickstoffdioxid zusätzlich in die Kolonne eingespeist. Nach üblicher Aufarbeitung wurde 2-Chlor-5-chlormethylpyridin in 72,4 % der Theorie erhalten. Als Nebenprodukt wurden 2,2 % 2-Chlor-5-hydroxymethylpyridin und 0,4 % 2,6-Dichlor-5-chlor-methylpyridin identifiziert. In der wässrigen Phase waren etwa 20 % der Theorie an 5-Chlormethylpyridon-2 vorhanden, welches auf bekannte Weise ebenfalls in 2-Chlor-5-chlormethylpyridin überführbar ist.

### Beispiel 3

Durch die Glaskolonne wurden 1 Stunde lang 8 l/h eines Chlorwasserstoff/Stickstoff-Gemisches (15:85) in eine auf 0°C temperierte Lösung von 8,5 g 1-Methyl-5-aminopyrazolcarbonsäureethylester in 100 ml Chloroform geleitet. Dann schaltete man 1 Stunde lang 8 l/h Stickstoffdioxid zu. Die Reaktionsmischung wurde im Vakuum eingeengt, in 100 ml konzentrieerter Salzsäure gelöst und anschließend eine Lösung von 31 g Kupfersulfat und 28 g Kochsalz in 100 ml Wasser zugetropft. Nach 1,5 Stunden Rühren bei 50°C extrahierte man mit Chloroform, trocknete die vereinigten Extrakte mit wasserfreiem Natriumsulfat und engte am Rotationsverdampfer ein. 1-Methyl-5-chlorpyrazol-4carbonsäureethylester wurde in 76,7 % der Theorie erhalten.

## Patentansprüche

1. Verfahren zur Herstellung von Nitrosylchlorid aus Chlorwasserstoff und Stickstoffdioxid im molaren Verhältnis von 0.4 bis 1 bei Temperaturen von 0 bis 100°C, wonach man die Reaktanden im Gleichstrom von unten nach oben durch einen Reaktor führt und das gebildete Nitrosylchlorid oben und die gebildete Salpetersäure unten am Reaktor entnimmt.

2. Verwendung von nach Verfahren gemäß Anspruch 1 erhältlichem Nitrosylchlorid bei Diazotierungen oder Chlorierungen von Aminopyridinen.
